# EUROPEAN PATENT APPLICATION

(11) **EP 1 964 846 A1**
(43) Date of publication of application: **03.09.2008**
(21) Application number: 07103140.5
(22) Date of filing: 27.02.2007
(51) Int. Cl.: C07H 23/00, C07H 15/26, A61K 49/10

(54) **Process for the preparation of contrast agents**

(71) Applicant: Bracco S.P.A., 20134 Milano (IT); Università di Trieste, 34127 Trieste (IT)
(72) Inventor: Uggeri, Fulvio, 20134 Milano (IT); Campa, Cristiana, 20134 Milano (IT); Paoletti, Sergio, 20134 Milano (IT); Flamigni, Anna, 20134 Milano (IT)
(74) Representative: Poletti, Marco

(57) **Abstract**

The present invention refers to diagnostically or radiotherapeutically useful compounds which are able to selectively bind to lectins, having formula (I) 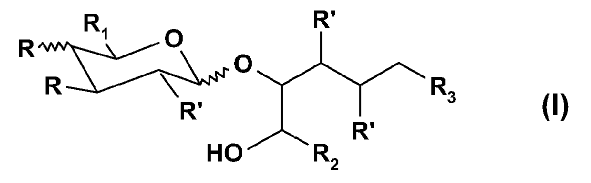 wherein R, R', R₁, R₂ and R₃ are as defined in the specification; the process for their preparation and the pharmaceutical compositions thereof.

## Description

The present invention relates to new compounds, which can chelate paramagnetic metal ions or radionuclides, the chelates thereof and their use as diagnostic contrast agents, particularly in Magnetic Resonance Imaging (hereinafter shortly referred to as M.R.I. or even as MRI) techniques, and as radiotherapeutic agents. More particularly, this invention refers to new diagnostically or radiotherapeutically effective agents comprising mono- and oligo-saccharide moieties covalently linked to the metal chelators.

From a radiologist's point of view, an improvement in the radiographic image, which means a better contrast enhancement between healthy and diseased tissues and organs, is certainly regarded as a valuable aid to the diagnosis itself. Means to the above image improvement may comprise, for instance, the administration of a suitable exogenous substance known as a contrast agent.

In MRI techniques, these substances may comprise paramagnetic complexes that are known to cause a significant alteration in relaxivity of the water protons belonging to the tissues under examination, whenever such protons are submitted to an external magnetic field.

Relaxivity is an intrinsic property of paramagnetic complexes which characterizes their ability to increase the nuclear magnetic relaxation rate of vicinal protons.

High relaxation rate may thus provide an increased contrast in the image quality, thus enabling the medical technicians to obtain physiological information in short times, with consequent advantages in terms of accuracy and costs.

Typically, MRI contrast agents include a suitably chelated paramagnetic metal ion. The paramagnetic metal ions most extensively used in MRI techniques are either in the transition metal series as well as in the lanthanide series.

As far as lanthanides are concerned, attention is mostly focused on gadolinium ion both for its paramagnets (7 unpaired electrons) and for its favourable properties in terms of electronic relaxation.

This metal ion is necessarily administered in a complexed form with a chelating ligand because of the strong toxicity of the free metal itself, e.g. of the gadolinium ion. The choice of the ligand is thus of crucial importance as it has to provide a thermodynamically and kinetically stable complex with the metal ion, whilst maintaining optimal relaxivity properties of the resulting gadolinium complex.

Chelated complexes of linear and cyclic polyaminopolycarboxylic ligands with paramagnetic metal ions as MRI contrast agents, thus including gadolinium ions, are widely known in the art.

Among them are, for instance, two well-known polyaminopolycarboxylic ligands, namely diethylenetriaminopentaacetic acid (DTPA) and 1,4,7,10-tetraacetic acid (DOTA). The stability of the complexes of such ligands with gadolinium is such to hamper the release of the very toxic Gd³⁺ free ions in the body. However, because of their molecular size, these complexes undergo rapid excretion following an extra cellular unspecific biodistribution.

For this reason, a number of investigations have recently focused on the binding of Gd-DTPA²⁻ and Gd-DOTA⁻ to biocompatible macromolecules that are able to increase target specificity of the gadolinium complexes [see, as a reference, R. Rebizak *et al.,* 1997].

Most of the commercially available MRI contrast agents, indeed, are able to give rise to a diagnostically effective signal but lack in biospecificity which, in its turn, could be important for dose reduction and/or improvement of image quality.

The biospecificity of gadolinium-based contrast agents could be achieved, for instance, upon suitably modifying the chemical structure of the ligand itself, for instance by coupling Gd-DTPA²⁻ with a monoclonal antibody [see, as a reference, D. Shahbazi-Gahrouei *et al.,* 2001].

This is not an easy task as an alteration of the gadolinium surroundings might determine a decrease in relaxivity which, however, could be counterbalanced by an increase in tissue specificity, thus allowing for lower doses of contrast agent to be administered and lower signal background.

The presence of specific saccharidic moieties on the cell surface of certain tissues or organs is commonly known. Furthermore, specific sugar-binding proteins (lectins) accomplish to a wide variety of roles (e.g. cell-cell and cell-matrix interactions) [see, as a reference, S. Asayama *et al.,* 2004; C. Burtea *et al.,* 2003; P. Marcon *et al.,* 2005; H.

Lis *et al.,* 1998; B.G. Davis, 2002; and R.S. Haltiwanger *et al.,* 1986]. The lectin-sugar interactions may thus be exploited in the diagnosis and/or therapy of pathologies involving the modification of the expression of lectins, upon designing specific carbohydrate-containing drugs [see, as a reference, J.P. André *et al.,* 2004].

Tumours are pathologies in which lectin variations may be involved. In particular, within the family of galactose binding lectins (galectins), an over-expression of galectin 3 is present in several neoplastic tissues; e.g. neoplastic follicular thyroid tissues [H. Inohara *et al.,* 1999] glioblastomas [G. Elad-Sfadia *et al.,* 2004], non-small cell lung cancers [F. Puglisi *et al.,* 2004], breast tumours [K. S. Khaldoyanidi *et al.,* 2003], pancreatic tumours [P.O. Berberat *et al.,* 2001] and colon adenocarcinomas [M.E. Huflejt *et al.,* 1997]. Galectin 3 is also involved in the exacerbation of rheumatoid arthritis [M. Neidhart *et al.,* 2005], while galectin 4 seems to be strongly correlated with inflammatory bowel diseases (i.e. ulcerative colitis and Chron's disease) [A. Hokama *et al.,* 2004].

Other quite typical interactions involve β-galactose: a receptor (ASGP-R), localized on the hepatocyte cell membrane, recognizes asialoglycoproteins bearing a terminal β-galactose [S. Asayama *et al.,* 2004, K. Kobayashi *et al.,* 1994]. In this context, Asayama and co-workers have demonstrated that, when Mn-porphyrin are conjugated with lactose, a 3-fold increase in cellular recognition is observed on HepG₂ cells, given the interaction between lactose and ASGP-Rs expressed on the surface of hepatic cells.

Moreover, some oligosaccharides are known to interact with specific enzymes which can be over- or under-expressed in some pathologies [S. Aime *et al.,* 2002 and reference therein]. In their review, Aime and co-authors report some applications of targeted paramagnetic probes: the use of Gd-HPDO3A containing a β-galactose moiety, which is subject to the action of β-galactosidase, deserves a special mention. Once the β-galactose is *in vivo* enzymatically removed from the contrast agent, the diagnostic signal is enhanced allowing the detection of an over-expression of β-galactosidase as, for example, in gene transfection.

Various attempts have been made to link biologically active sugars to ligands for paramagnetic cations [S. Asayama *et al.,* 2004, J.P. André *et al.,* 2004, C. Burtea *et al.,* 2003, S. Aime *et al.,* 2002]; such strategies involve several synthetic steps which are time-consuming and often imply structural modification on the saccharidic moiety itself, thus affecting the desired biological activity of the contrast agents.

Indeed, upon choosing reaction conditions that do not alter the biologically active core of the saccharidic moiety, it is possible to achieve metal complexes possessing the same bio-interaction capability of the linked carbohydrates.

The most used conjugation routes imply the formation of amidic bonds though, occasionally, thio-ether and ester bonds have been described [J.P. Andrè *et al.,* 2004, P.Baìa *et al.* 2005, US 4822594, EP 186947, EP 0707857, WO 99/01160].

We have now found specific diagnostically or radiotherapeutically effective agents that are characterized by high bioselectivity.

In particular, the present invention refers to novel derivatives comprising at least a saccharidic residue covalently linked to the metal chelator, otherwise referred to as ligand. When intended for diagnostic purposes, in particular as MRI contrast agents, the ligands thus modified provide an increased relaxivity, due to the hydrophilicity of the bound carbohydrates. In addition, because of the presence of the saccharidic moiety, the diagnostically or radiotherapeutically effective agents of the invention result to possess a particularly high bio-specificity.

The present invention regards simple, rapid and efficient strategies for the preparation of such compounds, whilst maintaining intact the bioactive structural motifs of the carbohydrates themselves.

Therefore, a first object of the present invention is represented by the compounds of formula (I) 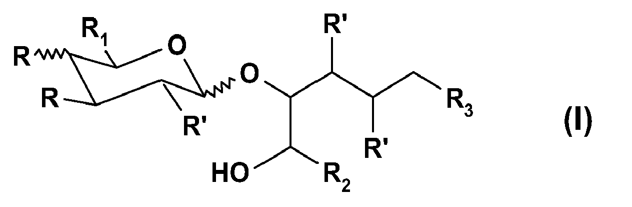 wherein:
- **R**: is, the same or different and each independently, a group selected from hydroxyl (-OH), acetate (-OCOCH₃), sulphate (-OSO₃H), phosphate (-OPO₃H₂), amino (-NH₂) or acetylamino (-NHCOCH₃); or one of the R groups is as above defined and the other is a straight or branched saccharidic or oligosaccharidic chain;
- **R'**: is, the same or different and each independently, a group selected from hydroxyl (-OH), acetate (-OCOCH₃), sulphate (-OSO₃H), phosphate (-OPO₃H₂), amino (-NH₂) or acetylamino (-NHCOCH₃);
- **R₁** and **R₂**: are, the same or different and each independently, hydroxymethyl (-CH₂OH), carboxyl optionally esterified with a straight or branched C₁-C₄ alkyl chain (-COOH, -COOAlk), a group of formula (II)

-CH₂-O-CH₂-CH(OH)-CH₂R₃ (II)

or a group -CH₂R₃;
- **R₃**: is, independently in each occurrence, a diagnostically or radiotherapeutically effective chelating moiety labelled with a paramagnetic metal ion or radionuclide, bearing a terminal amino group for its linkage (-NH-) with the rest of the molecule;
and the pharmaceutically acceptable salts thereof.

The compounds of the present invention may possess one or more asymmetric carbon atoms, otherwise known as chiral carbon atoms, and may thus exist in optically active or racemic form. Unless otherwise provided, the present invention encompasses any of the said compounds of formula (I) in optically active or racemic form as well as any mixture thereof.

Likewise, unless otherwise provided, the sugar ring or rings as per formula (I), thus comprising those derivatives bearing a saccharidic or oliogosaccharidic chain (R), may be present in any of the two alpha and beta anomeric conformations, both to be intended as comprised within the scope of the invention.

When referring to R, with the term saccharidic or oligosaccharidic chain we intend, unless otherwise provided, a moiety, residue or chain deriving from a monosaccharide, a disaccharide or an oligosaccharide, wherein the glycosidic units are in their biologically active form.

From the above, it is clear to the skilled person that glycosidic units in the biologically active form are those same units wherein the substituents onto the cyclic sugar ring or rings are properly and stereochemically oriented, thus enabling them to exert their biological action.

Preferably, the above saccharidic moieties are selected from the group consisting of lactitol (biologically active moiety: β-D-galactopyranoside), lactosylamine [biologically active moiety: N-(β-D-lactopyranoside)], maltitol (biologically active moiety: α-D-glucopyranoside), maltosylamine [biologically active moiety: N-(β-D-maltopyranoside)] or modified citrus pectins.

For a general reference to lactitol, lactosylamine, maltitol or maltosylamine see, as an example, "Essentials of carbohydrate chemistry and biochemistry", T.K. Lindhorst, ed. Wiley-WCH, 2000; for a general reference to pectins see, as an example, [P. Nangia-Makker *et al.,* 2002; D. Platt *et al.,* 1992].

According to an embodiment of the invention, preferred compounds of formula (I) are those wherein both R groups are hydroxyl or, alternatively, one of them is hydroxyl and the other is a saccharidic or oligosaccharidic chain as set forth above.

According to this latter class, even more preferred are the compounds wherein the monosaccharide or saccharidic chain have formula (III) below, hence encompassing any of the formulae from (IIIa) to (IIIh): 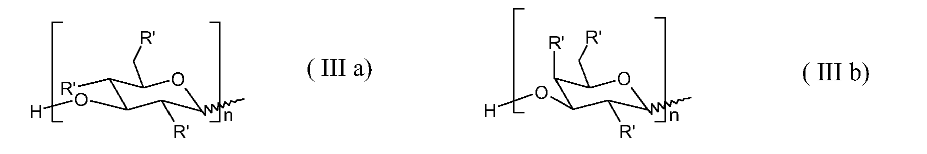 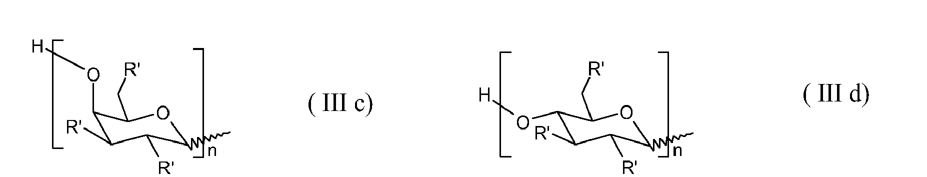 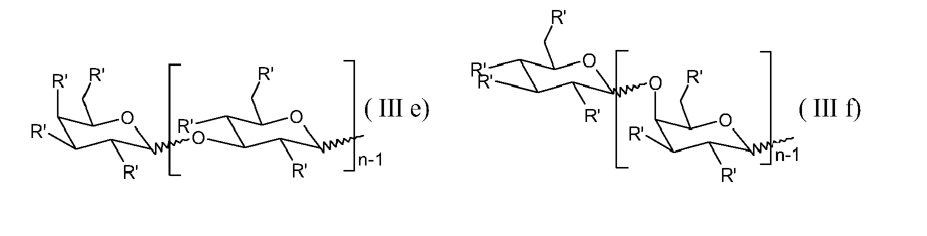 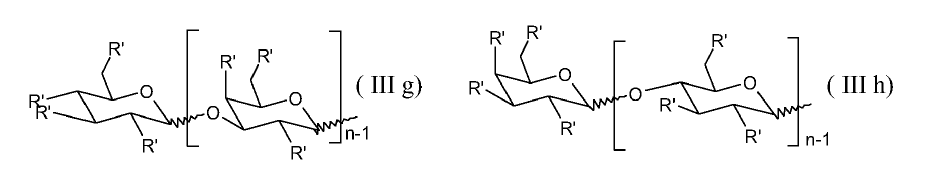 wherein R' is as above defined and n is an integer from 1 to 7.

According to this latter aspect, particularly preferred are the saccharidic or oligosaccharidic chains of formula (III) wherein n is an integer from 1 to 3.

As far as the R' groups are concerned, either in formula (I) as well as in any of the saccharidic or oligosaccharidic chains of formula (III), particularly preferred are those same groups as corresponding to hydroxyl.

With respect to the groups R₁ and R₂, in formula (I), they are the same or different and may correspond to hydroxymethyl (-CH₂OH), carboxyl optionally esterified with a straight or branched C₁-C₄ alkyl chain (-COOH, -COOAlk), a group of formula (II)

-CH₂-O-CH₂-CH(OH)-CH₂R₃ (II)

or a group -CH₂R₃.

Unless otherwise provided, with straight or branched C₁-C₄ alkyl chain we intend, methyl, ethyl, n-propyl, isopropyl, n-butyl, isobutyl, sec-butyl and tert-butyl.

As R₃ is the group bearing the chelating moiety, it is clear to the skilled person that according to the meanings provided to R₁ and R₂, the compounds of formula (I) of the invention may possess one or more of these chelating moieties.

Therefore, according to a first embodiment, when both groups R₁ and R₂ are hydroxymethyl or optionally esterified carboxyl, then a single chelating moiety is present in the compounds of formula (I).

According to a different embodiment, when one of R₁ or R₂ is hydroxymethyl or optionally esterified carboxyl and the remaining one is -CH₂-O-CH₂-CH(OH)-CH₂R₃ (II) or -CH₂R₃, two chelating moieties are present in the compounds of formula (I).

Likewise, according to a still different embodiment, when both of R₁ and R₂ are other than hydroxymethyl or optionally esterified carboxyl, namely they are any one of the groups -CH₂-O-CH₂-CH(OH)-CH₂R₃ (II) or -CH₂R₃, three chelating moieties are present in the compounds of formula (I).

Preferably, unless otherwise provided, when more than one chelating moiety is present in the compounds of the invention, that same moiety is the same at each occurrence.

As formerly reported, R₃ is a diagnostically or radiotherapeutically effective chelating moiety, properly labelled with a paramagnetic metal ion or radionuclide, bearing a terminal amino group for its linkage (-NH-) with the rest of the molecule.

This is to say that the compounds of the invention may comprise a chelating moiety ending with an amino group (-NH₂) or, alternatively, any other chelating moiety bearing a different functional group however susceptible of being properly modified so as to give rise to a chelating moiety bearing that same terminal amino group.

We refer, just as an example, to any of the well known chelating moieties (e.g. chelants or ligands) bearing a functional group other than amino (e.g. a carboxylic or phosphonic group) that can be properly reacted so as to lead to a different chelator bearing, through a spacer, the said terminal amino group.

Without posing any limitation, the above can be schematically represented as follows:
CHELANT-COOH + H₂N-Y-NH₂ → CHELANT-CONH-Y-NH₂ or
CHELANT-COOH + HO-Y-NH₂ → CHELANT-COO-Y-NH₂ or
CHELANT-OPO₃H₂ + OH-Y-NH₂ → CHELANT-OP(O)(OH)O-Y-NH₂ and the like,
wherein R₃ is respectively represented as:
CHELANT-CONH-Y-NH-,
CHELANT-COO-Y-NH-, or
CHELANT-OP(O)(OH)O-Y-NH-.

From the above, it is clear to the skilled person that Y, as a suitable spacer, may be represented by an amino acid, a peptide comprising from 2 to 4 amino acids, or by a straight or branched alkyl chain with from 1 to 10 carbon atoms, optionally interrupted by one or more groups selected from -O-, -CONH- or -NHCO-, -CO-, -COO-, -OCO- or -NR'- wherein R' is hydrogen or a straight or branched C₁-C₄ alkyl chain. Unless otherwise provided, with the term amino acid we refer to the natural amino acids in any of their optical configuration, hence including D or L amino acids; with the term peptide, instead, we refer to those same amino acids properly conjugated to each other through the formation of amido bonds.

More in particular, with respect to R₃ and unless otherwise provided, with the term diagnostically effective chelating moiety properly labelled with a paramagnetic metal ion or radionuclide, we refer to any moiety being detectable by imaging diagnostic procedures, that is to say any moiety able to provide, to improve or, in any way, to advantageously modify the signal detected by an imaging diagnostic technique.

The above techniques may thus include, for instance, magnetic resonance imaging (MRI) or radioimaging, thus enabling the registration of diagnostically useful, preferably contrasted, images when used in association with such techniques.

Examples of diagnostically effective moieties according to the invention include, for instance, paramagnetic metal ions in the form of chelated or polychelated complexes as well as chelated gamma ray or positron emitting radionuclides.

Clearly, it is well known to the skilled person that the imaging modality to be used needs to be selected according to the imaging detectable moiety the diagnostic compounds of the invention includes.

Unless otherwise provided, the terms "chelator", "chelating ligand" or "chelating agent", used herein interchangeably, refer to chemical moieties, agents, compounds, or molecules characterized by the presence of polar groups able to form a complex containing more than one coordinated bond with a transition metal or another metal entity. In a preferred aspect of the invention, the chelating ligands include cyclic or linear polyamino polycarboxylic or phosphonic acids and usually contain amino, thiol or carboxyl groups able to provide for the above complexation or labelling. The terms "labelled/labelling" or "complexed/complexation", in the present context, relate to the formation of a chelate or coordinated complex between the selected metal entity and the chelating ligand itself.

Said metal entities specifically include paramagnetic metal ions that are detectable by imaging techniques such as Magnetic Resonance Imaging (MRI), or to metal ions (e.g. radionuclides) that are detectable by imaging techniques such as scintigraphic imaging, Single Photon Emission Computed Tomography (SPECT) and Positron Emission Tomography (PET).

In a preferred embodiment of the invention, within the compounds of formula (I), R₃ is an MRI detectable moiety comprising a chelating ligand labelled with a paramagnetic metal ion detectable by Magnetic Resonance Imaging (MRI) techniques.

Preferred paramagnetic metal elements are those having atomic number ranging between 20 and 31, 39, 42, 43, 44, 49 and between 57 and 83. More preferred are paramagnetic metal ions selected from the following: Fe(2+), Fe(3+), Cu(2+), Ni(2+), Rh(2+), Co(2+), Cr(3+), Gd(3+), Eu(3+), Dy(3+), Tb(3+), Pm(3+), Nd(3+), Tm(3+), Ce(3+), Y(3+), Ho(3+), Er(3+), La(3+), Yb(3+), Mn(3+), Mn(2+) wherein Gd(3+) is the most preferred.

Suitable chelating ligands include those selected from the group consisting of: polyaminopolycarboxylic acids and derivative thereof, comprising, for example, diethylenetriamine pentaacetic acid (DTPA), benzo-DTPA, dibenzo-DTPA, phenyl-DTPA, diphenyl-DTPA, benzyl-DTPA, dibenzyl DTPA, N,N-bis[2-[(carboxymethyl)[(methylcarbamoyl)methyl]amino]ethyl]-glycine (DTPA-BMA), N-[2-[bis(carboxymethyl)amino]-3-(4-ethoxyphenyl)propyl)]-N-[2-[bis(carboxymethyl) amino]ethylglycine (EOB-DTPA), 4-carboxy-5,8,11-tris(carboxymethyl)-1-phenyl-2-oxa-5,8,11-triazatridecan-13-oic acid (BOPTA); N,N-bis[2-[(carboxymethyl)[(methylcarbamoyl)methyl]amino]ethyl]-glycine (DTPA-BMA); N,N-bis[2-[bis(carboxymethyl)amino]ethyl]L-glutamic acid (DTPA-GLU), DTPA conjugated with Lys (DTPA-Lys); ethylenediaminetetraacetic acid (EDTA); 1,4,7,10-teraazacyclododecane 1,4,7,-triacetic acid (DO3A); 1,4,7,10-tetraazacyclotetradecane-1,4,7,10-tetraacetic acid (DOTA); [10-(2-hydroxypropyl)- 1,4,7,10-teraazacyclododecane 1,4,7,-triacetic acid (HPDO3A); 6-[bis(carboxymethyl)amino]tetrahydro-6-methyl-1H-1,4-diazepine-1,4(5H)-diacetic acid (AAZTA) and derivatives thereof, as per the international patent application WO 03/008390 incorporated herein by reference; 2-methyl-1,4,7,10-tetraazacyclotetradecane-1,4,7,10-tetraacetic acid (MCTA); (α,α',α",α"')-tetramethyl-1,4,7,10-tetraazacyclotetradecane-1,4,7,10-tetraacetic acid (DOTMA); polyaminophosphate acid ligands or derivatives thereof including, in particular, N,N'-bis-(pyridoxal-5-phosphate)ethylenediamine-N.N'-diacetic acid (DPDP), ethylenedinitrilotetrakis(methylphosphonic) acid (EDTP); polyaminophosphonic acid ligands and derivatives thereof, for instance including 1,4,7,10-tetraazacyclotetradecane-1,4, 7,10-tetrakis [methylphosphonic)] and 1,4,7,10-tetraazacyclotetradecane-1,4,7,10-tetrakis[methylene-(methylphosphinic)] acid; macrocyclic chelators such as texaphyrins, porphyrins or phthalocyanines.

Preferred ligands according to the present invention include, for instance, DTPA-GLU, DTPA-Lys, AAZTA-Lys and derivatives thereof.

According to an even preferred embodiment of the invention, particularly preferred R₃ groups are reported below (the parenthesis are herewith intended solely to highlight the terminal amino group):

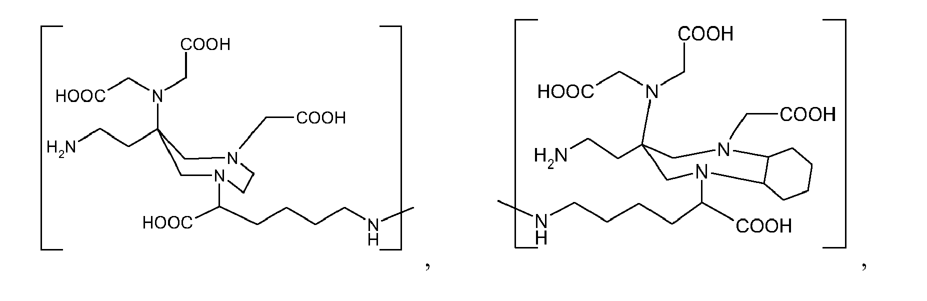

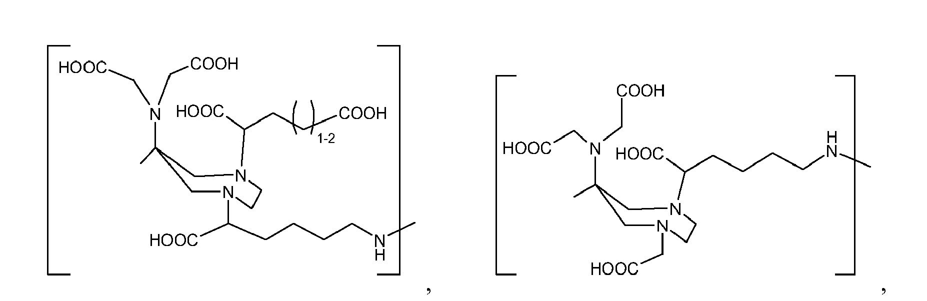

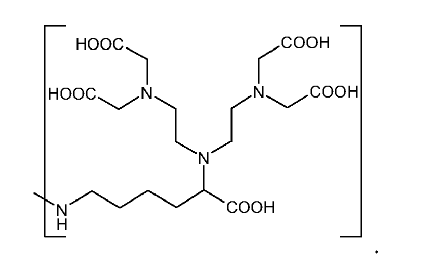

Examples of particularly preferred MRI contrast agents of the invention of formula (I), when complexed with a suitable paramagnetic metal ion, for instance gadolinium ion, may thus comprise:

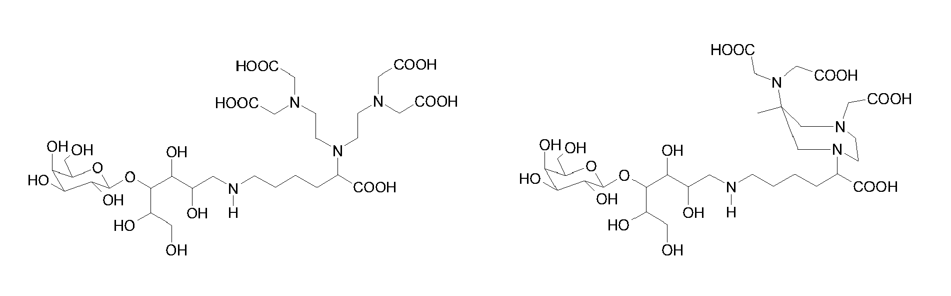

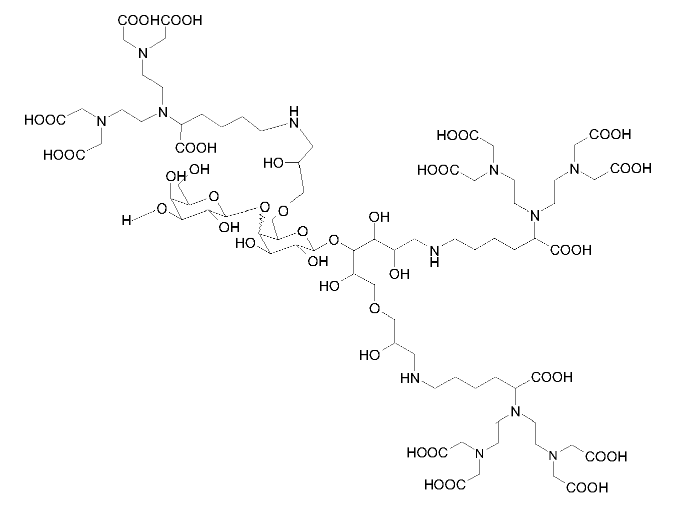

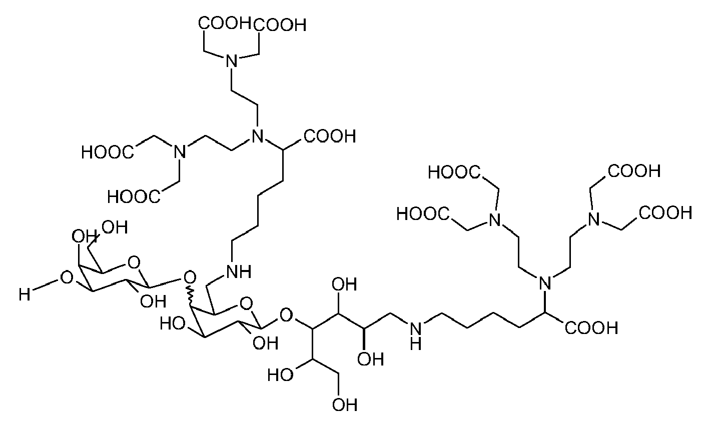

According to a different embodiment, the present invention also provides for novel compounds of formula (I) as radiographic contrast agents wherein R₃, as a diagnostically effective chelating moiety properly labelled with a radionuclide, is a radio imaging detectable moiety.

According to a still different embodiment, the present invention provides for novel compounds of formula (I) wherein R₃ is a chelating moiety properly labelled with a radionuclide having radiotherapeutical effect.

In both cases, that is for diagnostic (e.g. radiographic) or for therapeutic (e.g. radiotherapy) purposes, R₃ is a suitably chelated radionuclide.

More in particular, however, whilst a "radioimaging detectable moiety" refers to a radionuclide moiety that is detectable by imaging techniques such as scintigraphic imaging, Single Photon Emission Computed Tomography (SPECT) and Positron Emission Tomography (PET), a "radiotherapeutic" moiety comprises a radionuclide which is therapeutically effective.

In the present context and unless otherwise provided, within the compounds of formula (I) as radiographic contrast agents or as radiotherapeutically effective agents, R₃ may comprise any of the suitable chelating ligands labelled with any suitable radionuclide known in the art as being usable for the intended diagnostic purpose or, alternatively, for the intended therapeutic purpose.

Just as an example, chelating ligands for radionuclides besides those above reported for MRI techniques may also comprise linear, macrocyclic, terpyridine, N3S, N2S2 and N4 chelators (for a reference see, as an example, the ligands reported in US 5,367,080; US 5,364,613; US 5,021,556; US 5,075,099 and US 5,886,142) and other chelating ligands known in the art including, but not limited to, HYNIC, TETA and bisamino bisthiol (BAT) chelators (see also US 5,720,934).

As an example, N4 chelating ligands are described in US patent Nos. 6,143,274; 6,093,382; 5,608,110; 5,665,329; 5,656,254 and 5,688,487.

Likewise, some N3S chelators are described in PCT/CA94/00395, PCT/CA94/00479, PCT/CA95/00249 and in US Patent Nos. 5,662,885; 5,976,495 and 5,780,006. The chelators may also include derivatives of the chelating ligand mercapto-acetyl-acetyl-glycyl-glycine (MAG3), which contains an N3S and N2S2 system such as MAMA (monoamidemonoaminedithiols), DADS (N2S diaminedithiols), CODADS and the like. These ligand systems and a variety of others are described by Liu and Edwards; in Chem. Rev., 1999; 99, 2235-2268 and references therein.

The choice of the radionuclide will be determined based on the desired diagnostic or therapeutic application.

For therapeutic purposes, for instance so as to provide radiotherapy for primary tumors and metastasis, the known preferred radionuclides may include 64Cu, 90Y, 105Rh, 111In, 117mSn, 149Pm, 153Sm, 161Tb, 166Dy, 166Ho, 175Yb, 177Lu, 186/188Re, 199Au, and 159Gd, with 177Lu and 90Y being particularly preferred.

Instead, for diagnostic purposes, for instance to monitor therapeutic progress in primary tumors and metastases, the known preferred radionuclides may include 64Cu, 67Ga, 68Ga, 99mTc, and 111In.

99mTc is particularly preferred for diagnostic applications because of its low cost and availability, as it is readily available from a 99Mo-99mTc generator, and because of its optimal imaging properties and high specific activity.

In addition to the above, it is worth noting that the choice of the suitable ligand residue may also depend on the radionuclide being used for the ligand labelling.

Thus, for instance in the case of 99mTc, 186Re, and 188Re radionuclides, particularly preferred are the ligands described in US Patent Nos. 6,093,382; 5,608,110; 6,143,274; 5,627,286; 6,093,382; 5,662,885; 5,780,006; 5,627,286 and 5,976,495 which are incorporated by reference herein in their entirety.

Clearly, according to the present invention, any of the said chelating ligands (R₃): (i) for diagnostic purposes, properly labelled with a paramagnetic metal ion or with a radionuclide detectable through radioimaging techniques; or (ii) for therapeutic purposes, properly labelled with a radionuclide having radiotherapeutic activity; has to comprise a terminal amino group for its linkage with the rest of the molecule of formula (I) or it has to be properly modified with any suitable spacer, as set forth above, so as to comprise that same terminal amino group (-NH-).

As formerly reported, the compounds of formula (I) of the invention can also be present in the form of a pharmaceutically acceptable salt.

The term "pharmaceutically acceptable salt", as used herein, refers to derivatives of the compounds of the invention wherein the parent compound is suitably modified by converting any of the free acid or basic groups, if present, into the corresponding addition salt with any base or acid conventionally intended as being pharmaceutically acceptable.

Apart from being non-toxic, the corresponding salts of the compounds of formula (I) of the invention are also characterized by a high stability, including a physiological stability upon usage and administration, so as not to impair the pharmacological activity or, more in general, any of the pharmacokinetic and pharmacodinamic properties of the parent compound.

Suitable example of the said salts may thus include mineral or organic acid addition salts of basic residues such as amino groups, as well as mineral or organic basic addition salts of acidic residues such as carboxylic, phosphonic or sulphuric groups.

Preferred cations of inorganic bases which can be suitably used to prepare salts within the invention comprise ions of alkali or alkaline-earth metals such as potassium, sodium, calcium or magnesium. Preferred cations of organic bases comprise, inter alia, those of primary, secondary and tertiary amines such as ethanolamine, diethanolamine, morpholine, glucamine, N-methylglucamine, N,N-dimethylglucamine.

Preferred anions of inorganic acids which can be suitably used to salify the complexes of the invention comprise the ions of halo acids such as chlorides, bromides, iodides or other suitable ions such as sulphates.

Preferred anions of organic acids comprise those routinely used in pharmaceutical techniques for the salification of basic substances such as, for instance, acetate, succinate, citrate, fumarate, maleate or oxalate.

Preferred amino acids that can be suitably used to salify the complexes of the invention may also comprise, for instance, taurine, glycine, lysine, arginine, ornithine, aspartic and glutamic acid.

According to another embodiment, the present invention provides for a process for the preparation of the compounds of formula (I) which process comprises, essentially, a reductive amination reaction between a suitable saccharide and a given chelating agent.

The above process, comprehensive of any variant thereof as per the following details, is particularly advantageous as it enables the preparation of the compounds of the invention by working according to well known methods and operative conditions being adopted when carrying out reductive amination reactions.

Therefore, it is an additional object of the invention a process for the preparation of the compounds of formula (I) which process comprises:
a) reacting under reductive conditions, in a suitable solvent and in presence of an acidifying agent, a saccharide or oligosaccharide of formula (IV) 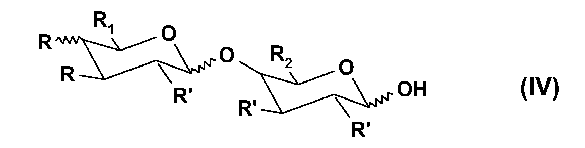 wherein R and R' are as above defined, and R₁ and R₂ are as set forth above or additionally represent, each independently, a aldehyde group (-CHO); with a compound of formula (V)

   **R₃-H** (**V**)

   wherein R₃ is the chelating moiety ending with the terminal amino (-NH-) group in unlabelled form, so as to obtain a compound of formula (I') 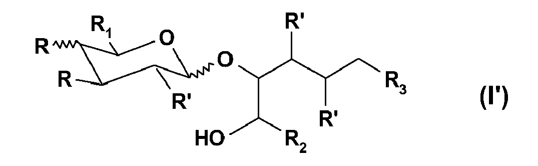 wherein R, R', R₁ and R₂ have the above reported meanings and R₃ is the above chelating unit; and
b) labelling the resulting compound of formula (I') with a paramagnetic metal ion or a radionuclide so as to obtain the compound of formula (I) and, optionally, converting it into a pharmaceutically acceptable salt thereof.

The present process is particularly advantageous as it allows preparing and recovering the desired compounds of the invention without altering the carbohydrate structure and, consequently, without impairing their biological properties.

The compounds of formula (I'), that is to say the compounds corresponding to formula (I) that are in a still unlabelled form or, in other words, wherein the chelating ligand has not been subjected yet to complexation with the selected paramagnetic metal ion or radionuclide, are novel and hence represent a further object of the invention.

As far as step (a) is concerned, the reaction between the compounds of formula (IV) and (V) is carried out one pot. Both reactants are first dissolved into suitable solvents and the resulting solutions are properly admixed or, alternatively, one of the said reactants is added to a suitable solution of the other reactant, in any order.

Preferably, suitable solvents may comprise water, lower alcohols, tetrahydrofuran, acetonitrile, dimethylformamide and the like.

Even more preferred solvents are water, methanol, ethanol and mixtures thereof.

According to a preferred though non limiting embodiment the saccharide or oligosaccharide of formula (IV) is first dissolved in water and the resulting solution is then added to a proper solution of the compound of formula (V), preferably dissolved in methanol or ethanol.

The acidifying agent is any agent conventionally employed to bring pH below 7, for instance in a range between 4 and 5. Preferred acidic agents are glacial acetic acid, formic acid and the like.

The above reaction between the compound of formulae (IV) and (V) is carried out under reductive conditions, hence in the presence of any conventional reducing agent known to be used in reductive amination reactions or under catalytic hydrogenation.

Preferably, the above reductive conditions comprise using sodium cyanoborohydride, sodium tetraborate, lithium aluminium hydride, sodium triacetoxyborohydride, α-picoline-borane and the like; sodium cyanoborohydride and sodium tetraborate being particularly preferred.

Alternatively, in the case of catalytic hydrogenation, the reaction is carried out in the presence of conventional catalysts including, for instance, Ni/Raney or optionally supported Pt or Pd based catalysts.

Once obtained, the resulting compounds of formula (I') may be isolated from the reaction environment and subsequently recovered and purified according to conventional methods commonly used in organic chemistry techniques such as, for instance, High Performance Liquid Chromatography (HPLC), Size Exclusion Chromatography (SEC) and dialysis.

The operative conditions being employed, depending upon the desired compound of formula (I') to be recovered and purified, are all within the ordinary knowledge of the skilled person.

According to step (b) of the process, the labelling or complexation between the compound of formula (I'), bearing the chelating moiety or moieties, and the selected paramagnetic metal ion or radionuclide may be accomplished according to conventional methods. As an example, for instance in the case of chelate complexes with Gd (III) ions, labelling may be carried out as reported by P. Baía *et al.,* 2005; P.L. Anelli *et al.,* 2002; Z. Lu *et al.,* 2004; S. Langereis *et al.,* 2004; and S. Langereis *et al.,* 2005.

Finally, optional salification of the compounds of formula (I) may be carried out by properly converting any of the free acidic groups (e.g. carboxylic, sulforic, phosphonic and the like) or free amino groups into the corresponding pharmaceutically acceptable salts.

In this case too, the operative conditions being employed for the optional salification of the compounds of the invention are all within the ordinary knowledge of the skilled person.

According to a different aspect, it is also clear to the skilled person that functional groups being present in any reactant and/or intermediate derivative the present process refers to, either per the above steps (a) and (b) as well as per any variant thereof, and which could give rise to unwanted side reactions and by-products, need to be properly protected before the reaction takes place. Likewise, subsequent deprotection of those same protected groups may follow upon completion of the said reactions.

In the present invention, unless otherwise indicated, the terms "protecting group", designates a protective group adapted to preserving the function of the functional group to which it is bound. Specifically, protective groups are used to preserve amino, hydroxyl or carboxyl functions. Appropriate protective groups may thus include, for example, benzyl, benzyloxycarbonyl, alkyl or benzyl esters, or other substituents commonly used for the protection of such functions, which are well known to those skilled in the art [see, for a general reference, T. W. Green; Protective Groups in Organic Synthesis (Wiley, N.Y. 1981)].

Selective protection and deprotection of any of the said groups, for instance including carboxyl, hydroxyl and amino groups, may all be accomplished according to very well known methods commonly employed in organic synthetic chemistry.

From all of the above, it is also clear that any of the groups within the compounds of formula (I) and intermediates thereof, herewith identifiable as derivatized groups such as, for instance, acetate, sulphate, phosphate, acetiylamino, esterified carboxy and the like, may all be prepared from the functional groups from which they derive, by working according to conventional methods.

The process of the present invention, also comprehensive of any of the previous reactions concerning protection and deprotection steps, labelling with metal entities and optional salification, may be suitably modulated, as per the variants set forth below, so as to provide very efficient synthetic pathways for the preparation of specific compounds of formula (I) of the invention.

In this respect, a preferred synthetic approach for the preparation of a representative compound of the invention, for instance of a compound of formula (I) wherein the chelating moiety R₃ is present only once, that is to say that R₁ and R₂ do not rely on groups bearing additional R₃ groups, is reported in the following synthetic schemes 1 and 4.

Likewise, for instance in the case of the preparation of a representative compound of formula (I) wherein both of R₁ and R₂ have formula (II), that is to say that three chelating moieties are present in the final compounds, a preferred synthetic approach is reported in the following synthetic scheme 2.

This same pathway, in addition, provides for the preparation of compounds of formula (I) wherein one of the R groups is a saccharidic or oligosaccharidic chain.

The following scheme 3, instead, refers to a possible synthetic approach for the preparation of a representative compound of formula (I) wherein R₁ is a group -CH₂R₃ (e.g. two R₃ chelating units in the final compounds).

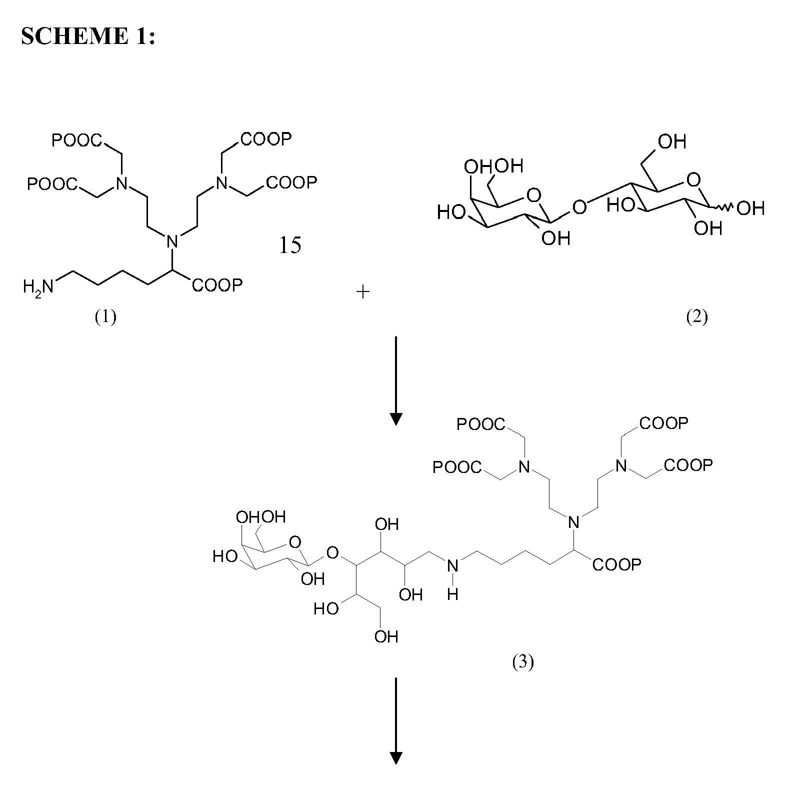

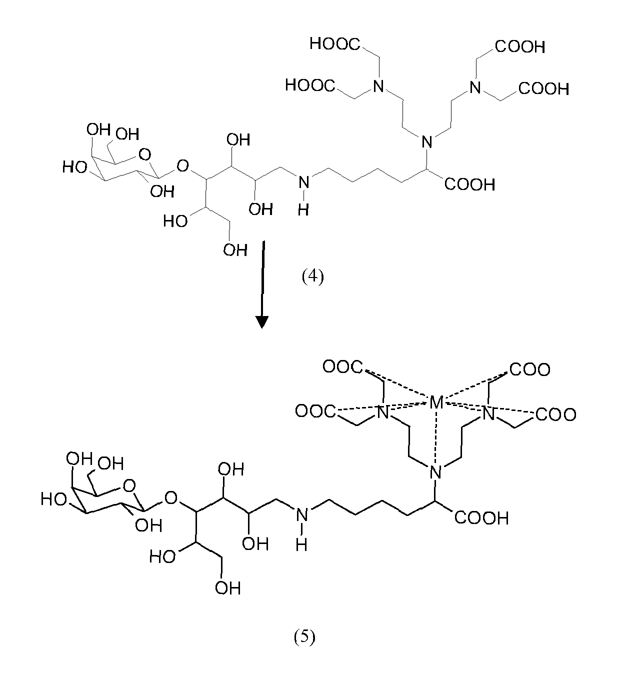

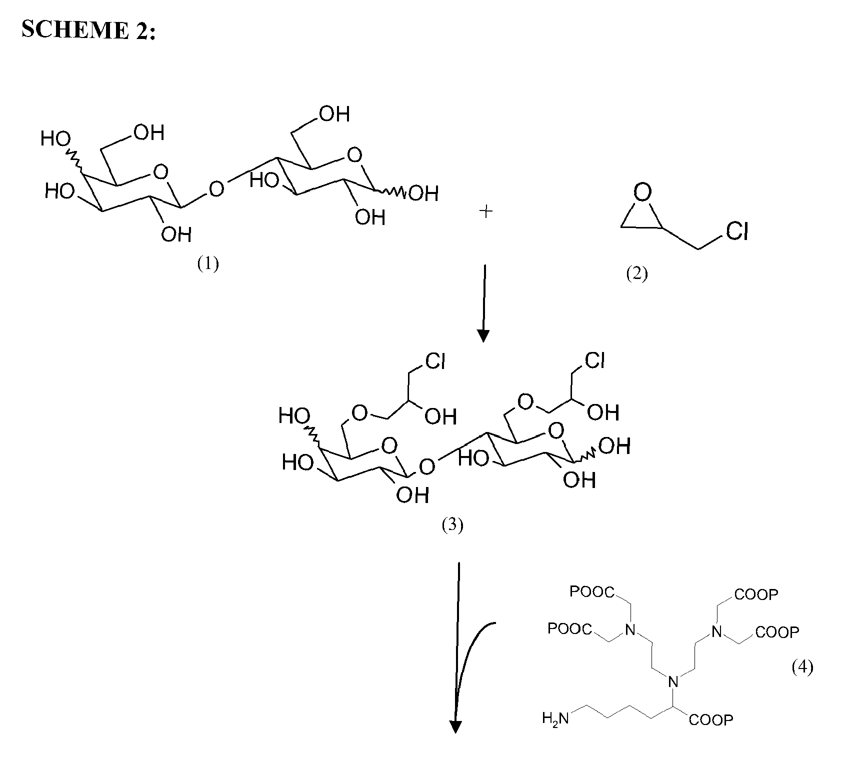

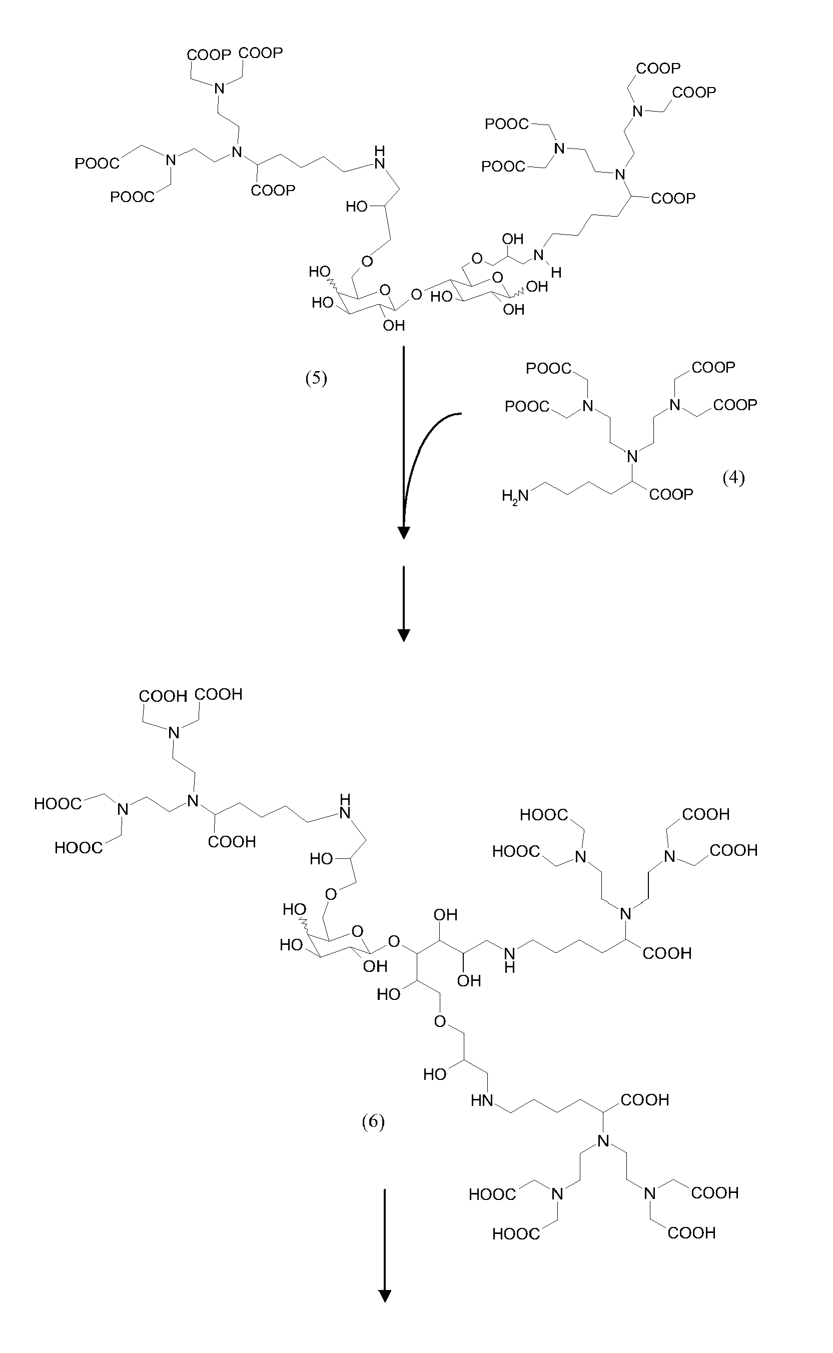

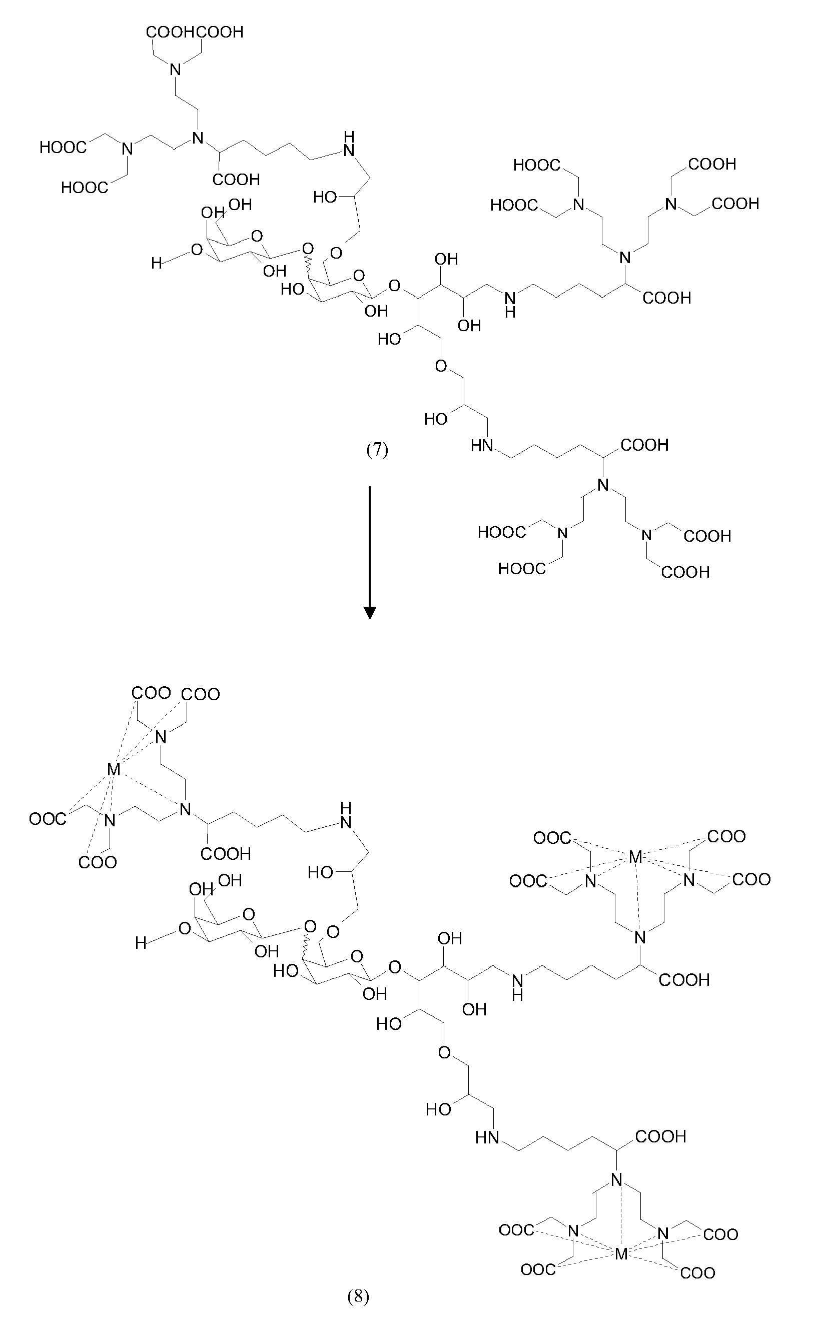

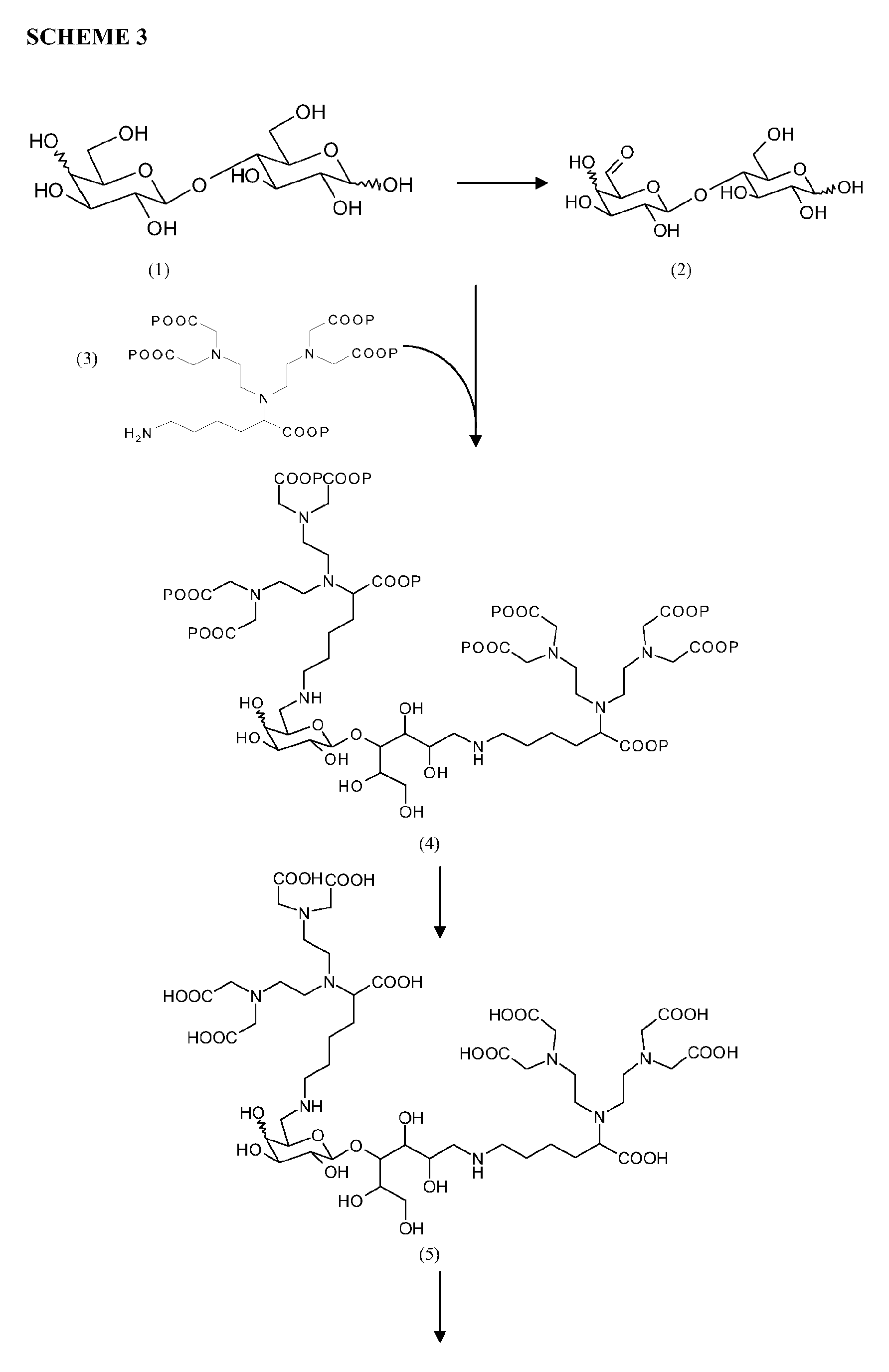

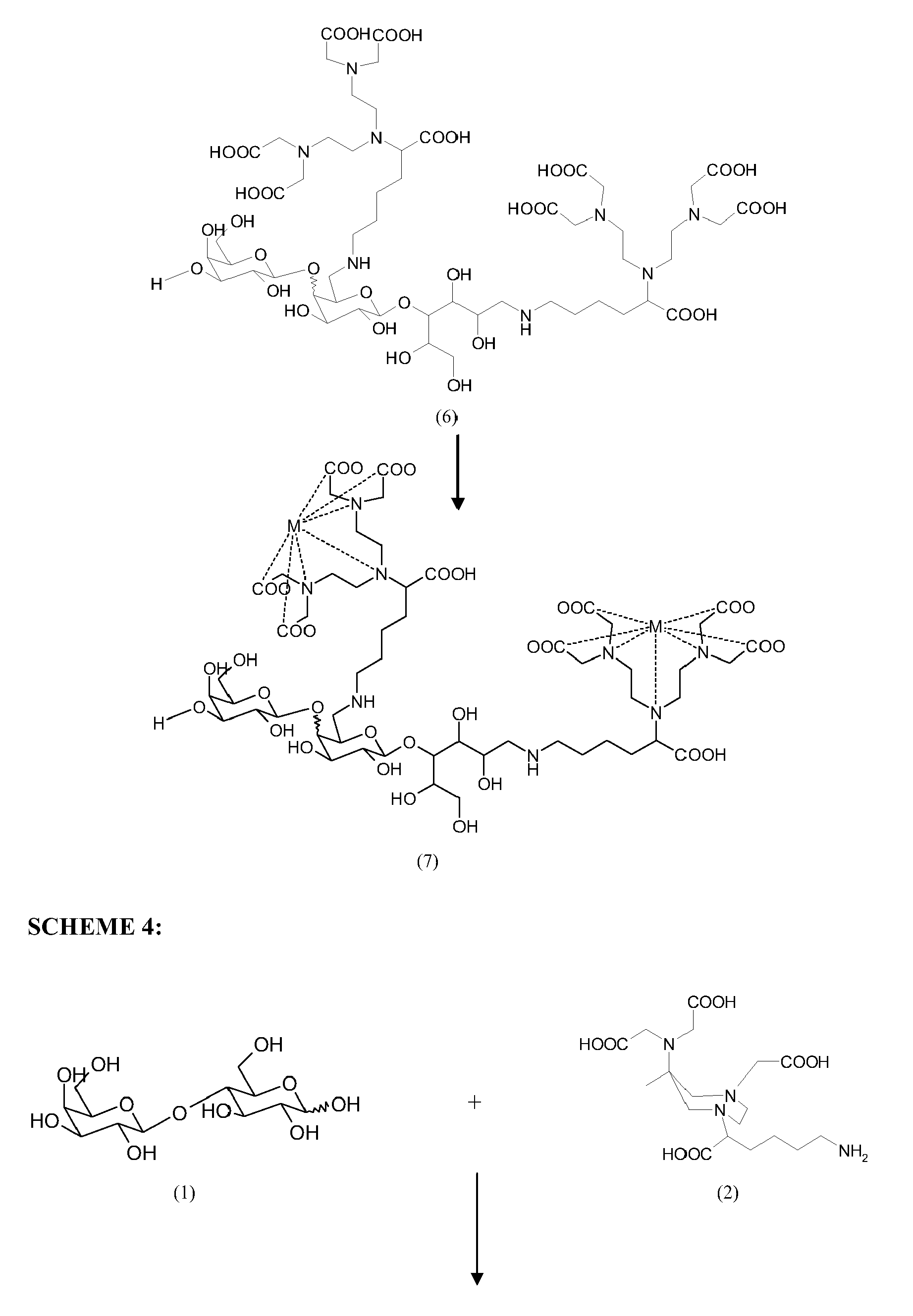

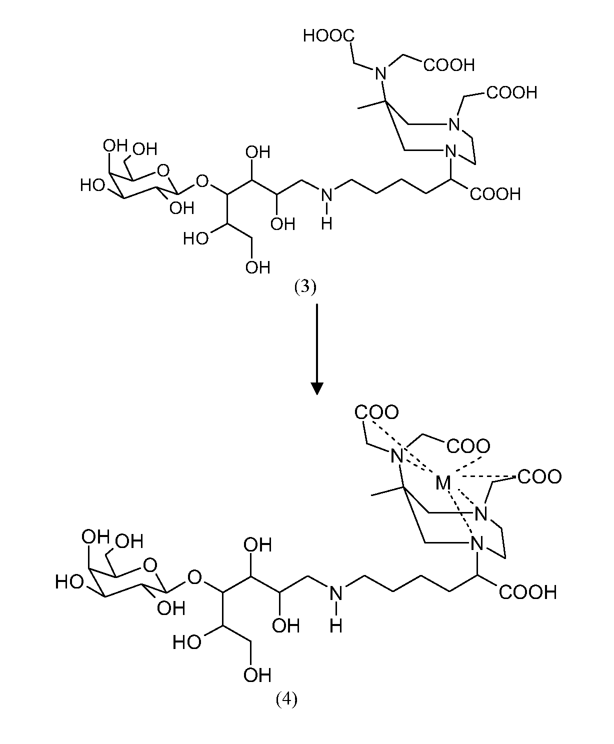

Unless otherwise provided, in the above synthetic pathways, P may represent any suitable protecting group for the carboxyl function.

Deprotection may follow conventional methods including, for instance, basic or acidic treatment. Preferably, acidic treatment is performed in the presence of trifluoroacetic acid (TFA) in any suitable organic solvent (e.g. dichloromethane or chloroform).

The reductive amination reaction between the intermediates of formula (IV) and (V) is carried out as per step (a) of the process, by working according to conventional methods in the presence of a suitable reducing agent; reaction completion is afforded by keeping the reaction mixture in a screwed-cap vial at about 55°C for 6 hours.

The operative conditions being used for reductive amination and deprotection are carried out according to known methods, for instance as reported by [C. Burtea *et al.,* 2003]. Importantly, however, an amino bond (-NH-) rather than an amido bond is formed between the chelating ligand and the rest of the molecule.

As far as the elongation of the saccharidic chain is concerned (see schemes 2 and 3), it can be conveniently carried out chemically or enzymatically so as to maintain the expected biological activity of the final compound thus prepared (i.e. interaction with lectins). For a general reference to the operative conditions being adopted in the enzymatic reaction see, as a reference, [C.J. Hamilton, 2004].

With respect to scheme 3, therein provided is the insertion, either chemically or enzymatically, of an aldehydic or carboxylic group in C-6 [see compounds of formula (IV) of the process of the invention wherein R₁ and R₂ may also represent those same functional groups].

The above reaction may be carried out according to known methods including, for instance, the enzymatic oxidation of a corresponding hydroxymethyl group by means of a suitable enzyme (e.g. galactose oxidase). See, for a general reference, [G.Avigad *et al.,* 1985 - Protocol Amplex®Red,
http://probes.invitrogen.com/media/pis/mp22179.pdf]. Alternatively, that same selective oxidation can be achieved chemically by operating, for instance, as reported in [M.F.Semmelhack et al., 1984] or in US 6831173.

From all of the above, it is clear to the skilled person that the compounds of the invention may be all prepared according to several variants of the process comprising, essentially, the reaction between a compound of formula (IV) with a compound of formula (V), all of which are to be intended as within the scope of the invention.

The starting material and any reactant of the present process are known or can be easily prepared according to known methods.

More in particular, the saccharides or oligosaccharides of formula (IV) are commercially available or can be obtained as above reported from those commercially available.

Likewise, the chelating agents of formula (V) are known or can be easily prepared according to known methods. We refer, in particular, to those agents having the required terminal amino group like, for instance, DTPA-Lys or AAZTA-Lys and, also, to those known agents that can be suitably modified so as to bring that amino terminal group.

For a general reference to them and to their preparation, in particular of DTPA-Lys and AAZTA-Lys see, as an example, Anelli et al., in Bioconjugate Chem. 1999, 10, 137-140; and WO 2006/002873.

As formerly reported, tumours are pathologies in which lectin variations may be involved. As the compounds of the invention are able to selectively bind to and target lectins, they can be advantageously used to selectively localize diagnostically or radiotherapeutically effective moieties, for instance to solid tumours or metastatic tissues and organs.

The compounds of the present invention may thus find advantageous application for the diagnosis, prevention and treatment of all pathological conditions associated with an altered expression of lectins. Moreover, they may be advantageously employed to follow up and monitor oncological therapy efficacy and tumor treatment results.

According to an additional embodiment, therefore, the present invention provides for a compound of formula (I) for use as a diagnostic agent or radiotherapeutic agent.

Additionally, also comprised within the scope of the invention is the use of a compound of formula (I) in the preparation of a diagnostic or radiotherapeutic agent selectively binding to lectins.

In a still another embodiment, the invention concerns pharmaceutical compositions comprising, as an active ingredient, at least one compound of formula (I), including pharmaceutically acceptable salts thereof, in combination with one or more pharmaceutically acceptable carriers or excipients.

In a yet another aspect, the invention provides a method for imaging solid tumours or tumour cells, the method comprising the administration to a mammal under investigation of a diagnostic agent of the invention.

Furthermore, the invention provides a method for treating solid tumours comprising the administration to a mammal in need thereof of a radiotherapeutically effective amount of radiotherapeutic agent of the invention.

As an example, when the compounds of the invention are properly labelled with therapeutic radionuclides, they can find application either as radiopharmaceutical that will be used as a first line therapy in the treatment of a disease such as cancer or, alternatively, in combination therapy where the radiotherapeutic agents of the invention could be utilized in conjunction with adjuvant chemotherapy.

From all of the above it can be easily envisaged that the compounds of this invention may have a wide range of applications, since they can be used for intravasal, intrathecal, intraperitoneal, intralymphatic and intracavital administrations. Furthermore, the compounds are suitable for oral and enteral administration.

For the parenteral administration they can preferably be formulated as sterile aqueous solutions or suspensions, whose pH can range between 6.0 and 8.5.

The compounds of the present invention can optionally be chemically conjugated to suitable macromolecules or inglobated into suitable carriers.

With the aim of better illustrating the present invention, without posing any limitation to it, the following examples are now given.

### EXPERIMENTAL SECTION

### Example 1

### Synthesis and purification of a compound (I) wherein R is a hydroxyl group, both R₁ and R₂ are hydroxymethyl groups and R₃ is the DTPA-Lys moiety, having formula (ammonium as counter ion)

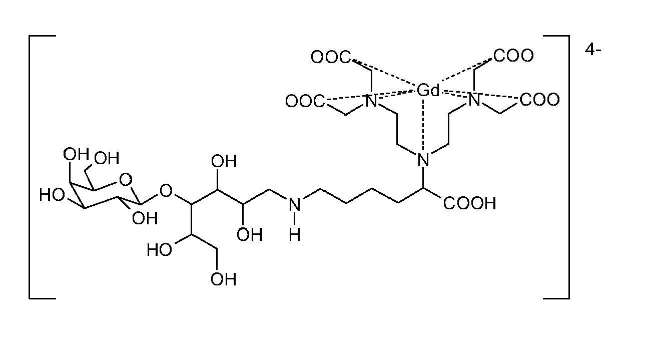

### Reductive amination between lactose and fully protected DTPA-Lys (see scheme 1)

0.5 mmol of lactose were dissolved in 0.5 mL of water and added to a mixture containing terbutylated DTPA-Lys (1.34 mmol) dissolved in methanol (6.266 mL), 0.733 mL of glacial acetic acid and 70 mg of sodium cyanoborohydride. The resulting solution was kept in a screwed-cap vial at 55 °C for 5 hours.

Crude reaction mixture purification was achieved by HPLC, by using a normal phase amino column. The pure product was eluted by isocratic acetonitrile/water (90/10). The system was connected to a fraction collector to allow automation and the pure fractions were brought to dryness by means of rotary evaporation (0.272 g, 50.8% yield).
¹H NMR (600 MHz; CD₃OD): δ = 4.51 (1 H; H-1), 4.19 (1 H; H-6'a), 3.9 (1 H; H-6'b), 3.88 (7 H; H-4, H-5, H-6a, H-6b, H-3', H-4', H-5'), 3.67 (1 H; H-3), 3.55 (2 H; H-2, H-2'), 3.45 (4x2 H; N*CH₂C(O)O),* 3.35 (2 H; H-4', NHCH₂CH₂CH₂CH₂*CH*C(O)O), 3.31 (3 H; H-1'a, H-1'b, NH*CH*₂CH₂CH₂), 2.8 (8 H; 2 x N*CH*₂CH₂N, 2 x NCH₂*CH*₂N), 1.78 (2 H; NHCH₂CH₂CH₂*CH*₂CHC(O)O), 1.5 (2 H; NHCH₂*CH*₂C*H*₂CH₂CHC(O)O), 1.52 (47 H; 45 x *tBu,* NHCH₂CH₂*CH*₂CH₂CHC(O)O). (Gal-unit: H-1-H-6b; Glc-ol-unit: H-1'-H-6'b).

### Deprotection

0.254 mmol of the fully protected compound from the previous step were re-suspended in 0.836 mL of dichloromethane. 4.7 mL of trifluoroacetic acid (TFA) were added to the suspension, at 0°C, and the solution was kept under stirring at room temperature for 48 hours. The solvent was then removed under reduced pressure and the product was washed with toluene (5 x 5 mL) yielding a thick light yellow foam that was re-suspended in milli-Q water and loaded onto a Superdex column eluted with water. The pure fractions were collected and concentrated so as to give the title compound in unlabelled form (51.3 mg, 25.6% yield).

### Gadolinium complexation

A 0.05 M aqueous solution of Gadolinium chloride (ligand/GdCl₃ molar ratio as 1:1) was slowly added while keeping pH between 6 and 7 by adding a 0.1 N solution of NH₄OH. After 2 h the solution was concentrated and loaded onto a Superdex column. A colorimetric test by means of Xylenol Orange for the determination of free gadolinium was performed on the relevant fractions eluted with water. Those resulting negative to the test were liofilized giving a yield of 61 %.

Relaxivity values of the title compound (I) are reported in table 1

**Table 1 Relaxivity values of compound (I)**

| | **r₁** | **r₂** |
|---|---|---|
| NaCl solution O.15M, pH 7.3 | 5.02 | 8.93 |
| Human serum 0÷1 mM | 6.9 | 11.7 |

### EXAMPLE 2

### Synthesis and purification of a compound (I) wherein R is a hydroxyl group, both R₁ and R₂ are hydroxymethyl groups and R₃ is the DTPA-Lys moiety, having formula (ammonium as counter ion)

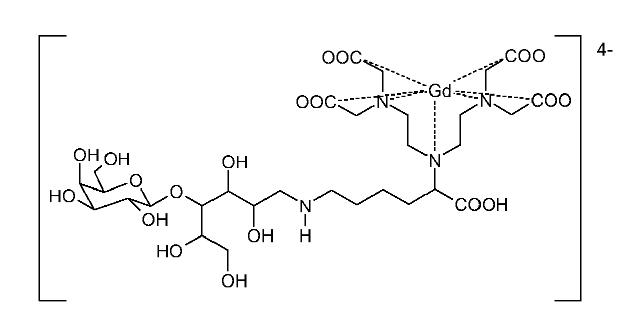

### Deprotection of DTPA-Lys

1.34 mmol of terbuthylated DTPA-Lys were dissolved in 10 mL TFA at 0°C and the solution was kept under stirring at room temperature for 48 hours. The product was co-distilled with toluene (5 x 10 mL). The product was brought to dryness by rotary evaporation, dissolved in milli-Q water and loaded onto a Superdex column eluted with water. The pure fractions were pooled and concentrated (343.36 mg, 55.22% yield).

### Reductive amination between lactose and fully deprotected DTPA-Lys (see scheme 1)

0.274 mmol lactose were dissolved in 1 mL of water and added to a mixture containing deprotected DTPA-Lys (0.74 mmol) dissolved in methanol (3 mL), 0.350 mL of glacial acetic acid and 70 mg of sodium cyanoborohydride. The resulting solution was kept in a screwed-cap vial at 55 °C over-night.

The mixture was brought to dryness by rotary evaporation and dissolved in 5 mL of water. The resulting solution was loaded onto a Superdex column and the product was eluted with milli-Q water. The pure fractions were pooled and concentrated (47 mg, 22% yield).

### Gadolinium complexation

A 0.05 M aqueous solution of Gadolinium chloride (ligand/GdCl₃ molar ratio as 1:1) was slowly added while keeping pH between 6 and 7 by adding a 0.1 N solution of NH₄OH. After 2 h the solution was concentrated and loaded onto a Superdex column. A colorimetric test by means of Xylenol Orange for the determination of free gadolinium was performed on the relevant fractions eluted with water. Those resulting negative to the test were liofilized giving a yield of 61 %.

### EXAMPLE 3

### Synthesis and purification of a compound (I) wherein R is a hydroxyl group, both R₁ and R₂ are hydroxymethyl groups and R₃ is the AAZTA-Lys moiety, having formula (ammonium as counter ion)

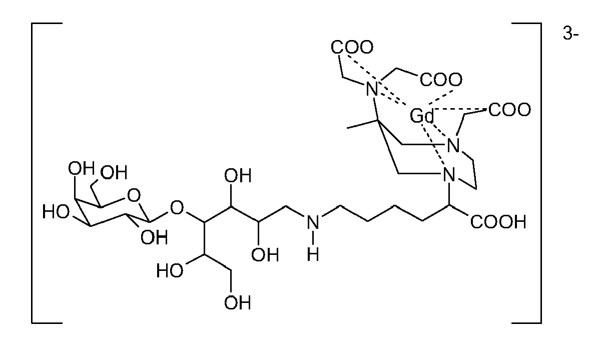

### Deprotection of AAZTA-Lys

0.3 mmol of terbuthylated AAZTA-Lys were dissolved in 1.54 mL of TFA at 0°C and kept under stirring at room temperature for 48 hours.

The crude reaction mixture purification was achieved by HPLC, using a phenylic column (10 µm, 10 x 250 mm) eluted with methanol/ammonium acetate (5 mM, pH 4.5) (table 2).

**Tabella 2: HPLC method**

| | | | | | | | |
|---|---|---|---|---|---|---|---|
| Time (min.) | **0** | **3.0** | **7.0** | **12.0** | **20.0** | **25.0** | **26.0** |
| **% A** | **97** | **97** | **40** | **10** | **10** | **97** | **97** |
| **% B** | **3** | **3** | **60** | **90** | **90** | **3** | **3** |

wherein:
A: CH₃COONH₄/CH₃COOH, pH 4.5, 5mM
B: CH₃OH

The pure fractions were pooled and liophylized twice in order to remove the acetate salts (93.5 mg, 72% yield).

Purification may also be achieved as previously described in example 2. Briefly, after removal of toluene, the product is resuspended in milli-Q water and loaded onto a Superdex column eluted with water. The pure fractions were pooled and concentrated (61% yield). Despite a 10% yield decrease, this purification method is preferred when scaling up.

### Reductive amination between lactose and fully deprotected AAZTA -Lys

0.063 mmol of lactose were dissolved in 0.189 mL of water and added to a mixture containing deprotected AAZTA-Lys (0.15 mmol) dissolved in methanol (1.67 mL), 0.066 mL of glacial acetic acid and 70 mg of sodium cyanoborohydride. The resulting solution was kept in a screwed-cap vial at 55 °C over-night.

The reaction mixture was brought to dryness and dissolved in water. The solution was filtered through a 0.2 µm filter. Purification was achieved upon loading the solution onto a Superdex column and the product was eluted with water (34.73 mg, 0.046 mmol, 73% yield).

### Gadolinium complexation

A 0.05 M aqueous solution of Gadolinium chloride (ligand/GdCl₃ molar ratio as 1:1) was slowly added while keeping pH between 6 and 7 by adding a 0.1 N solution of NH₄OH. After 2 h the solution was concentrated and loaded onto a Superdex column. A colorimetric test by means of Xylenol Orange for the determination of free gadolinium was performed on the relevant fractions eluted with water. Those resulting negative to the test were liofilized giving a yield of 60%.

By working in an analogous way, as per any one of examples from 1 to 3, and by selecting any proper saccharidic moiety and chelating agent, the following compounds may be obtained:

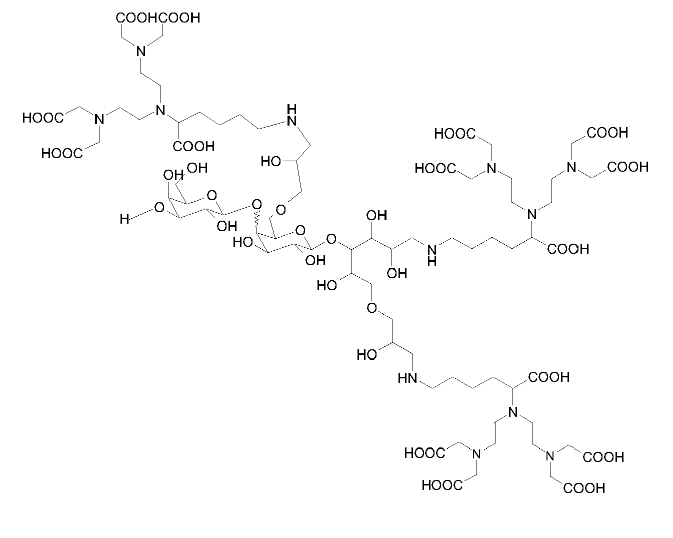

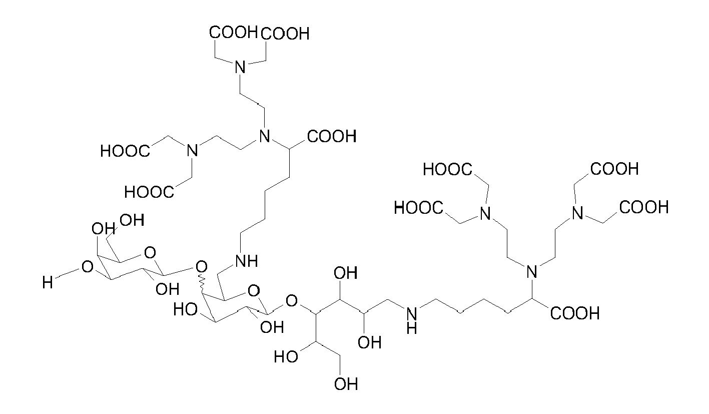

### References:

- S. Aime, C. Cabella, S. Colombatto, S.Geninatti Crich, E. Gianolio, F. Maggioni, Insights into the use of paramagnetic Gd(III) complexes in MR-molecular imaging investigations, Journal of magnetic resonance imaging, 16 (2002) 394-406
- S. Aime, M. Fasano, E. Terreno. Lanthanide(III) chelates for NMR biomedical applications Chem. Soc. Rev., 27(1998), 19-29 (1998)
- J.P. André, C. F. G. C. Geraldes, J. A. Martins, A. E. Merbach, M. I. M. Prata, A. C. Santos, J. J. P. De Lima, E. Tóth, Lanthanide (III) complexes of DOTA-glycoconjugates: a potential new class of lectin-mediated medical imaging agents, Chem. Eur. 10 (2004) 5804-5816
- P. L. Anelli, M. Brocchetta, P. Morosini, D. Palano, G. Carrea, L. Falcone, P. Pasta, D. Sartore. Conjugates of Gd(III) complexes to di- and tri-hydroxy substituted cholanoic acids: Regioselective oxidation with hydroxysteroid dehydrogenases. Biocatalysis and biotransformation, 20(1) (2002) 1024-2422
- S. Asayama, K. Mizushima, S. Nagaoka, H. Kawakami, Design of metallo porphyrin-carbohydrate conjugates for new superoxide dismutases mimic cellular recognition, Bioconjugate Chem, 15 (2004), 1360-1363
- G. Avigad. Oxidation rates of some desialylated glycoproteins by galactose oxidase. Archives of biochemistry and biophysics , vol. 239, no2, pp. 531-537 (1985)
- P. Baia, J. P. André, C. F. G. C. Geraldes, J. A. Martins, A. E. Merbach, E. Tóth, Lantanide (III) chelates of DTPA Bis(amide)Glycoconjugates: potential imaging agents targeted at the asyaloglycoprotein receptor, Eur. J. Inorg. Chem. (2005) 2110-2119
- P. O. Berberat, H. Friess, L. Wang, Z. Zhu, T. Bley, L. Frigeri, A. Zimmermann, and M. W. Büchler J. Histochem. Cytochem., 49 (2001) 539-549
- C. Burtea, S. Laurent, J. M. Colet, L. Vander Elst, R. N. Muller, Development of new glucosilated derivatives of gadolinium diethylenetriaminepentaceticacid for magnetic resonance angiography, Investigative radiology, 38 (2003), 320-333
- B. G. Davis, Synthesis of glycoproteins, Chem. Rev., 102 (2002), 579-601
- G.Elad-Sfadia, R.Haklai, Balan,E. and Y. Kloog, Biochem. Biophys. Res. Commun,. 325, (2004) 1393-1398
- R. S. Haltiwager, R. L. Hill, The ligand binding specificity and tissue localization of rat alveolar macrophage lectin, J. Biol. Chem., 261 (1986) 15696-15702
- C.J. Hamilton. Enzymes in preparative mono- and oligo-saccharide synthesis. Natural product reports, (2004), vol. 21, n°3, pp. 365-385
- A. Hokama, E. Mizoguchi, K. Sugimoto, Y. Shimomura, Y. Tanaka, M. Yoshida, S. T. Rietdijk, Y. P. de Jong, S. B. Snapper, C. Terhorst, R. S. Blumberg and A. Mizoguchi, Immunity, 20, (2004) 681-693
- M. E. Huflejt, E. T. Jordan, M. A. Gitt, S. H. Barondes, H. Leffler, J. Biol. Chem., 272, 22 (1997) 1429-14303
- H. Inohara, Y. Honjo, T. Yoshii, S. Akahani, J. Yoshida, K. Hattori, S. Okamoto, T. Sawada, A. Raz, Takeshi Kubo, Cancer, 85 (1999) 2475-2484
- K. S. Khaldoyanidi, V. V. Glinsky, L. Sikora, A. B. Glinskii, V. V. Mossine, T. P. Quinn, G. V. Glinsky and P. Sriramarao, J. Biol. Chem., 278, 6 (2003) 4127-4134
- K Kobayashi, A. Kobayashi, T. Akaike, Culturing hepatocytes on lactose-carrying polystyrene layer via asialoglycoprotein receptor-mediated interactions. In Methods in Enzymology; Y. C.Lee, R. T. Lee, Eds.; Academic Press: San Diego, CA, 1994; Vol. 247, p. 409-18.
- S. Langereis, A. Dirksen, B.F.M. de Waal, M.H.P. van Genderen, Q. de Lussanet, T.M. Hackeng, E.W. Meijer. Solid phase synthesis of a cyclic NGR-functionalized MRI contrast agent for target-specific imaging of angiogenesis European Journal of Organic Chemistry, 2005(12), 2534-2538, (2005)
- S. Langereis, Q.G. de Lussanet, M.H.P. van Genderen, W.H. Backes, T.M. Hackeng, J.M.A. van Engelshoven, E.W. Meijer, Towards target-specific molecular imaging of angiogenesis with Gd-DTPA-based dendritic architectures, Pol.Mater.Sci.Eng., 2004(91), 56, (2004)
- T.K. Lindhorst. "Essentials of carbohydrate chemistry and biochemistry", ed. Wiley-WCH, (2000)
- H. Lis, N. Sharon, Lectins: carbohydrate-specific proteins that mediate cellular recognition, Chem. Rev., 98 (1998) 637-674
- Z. Lu, D.L. Parker, K.C. Goodrich, X. Wang, J.G. Dalle, H.R. Buswell. Extracellular biodegradable macromolecular gadolinium(III) complexes for MRI. Magn Reson Med. (2004) 51:27-34.
- P. Marcon, E. Marsich, A. Vertere, P. Mozetic, C. Campa, I. Donati, F. Vittur, A. Gamini, S. Paoletti, Galectin-1 mediates interactions between chondhrocytes and a lactose-modified chitosan, Biomaterials, 26 (2005) 4975-84.
- P. Nangia-Makker, V. Hogan, Y. Honjo, S. Baccarini, L. Tait, R. Bresalier, A. Raz, Inhibition of human cancer cell growth and metastasis in nude mice by oral intake of modified citrus pectin. Journal of the National Cancer Institute, vol.94, n24, 1854-1862 (2002)
- M. Neidhart, F. Zaucke, R. von Knoch, A. Jüngel, B. A. Michel, R. E. Gay, S. Gay, Ann. Rheum Dis, 64 (2005) 419-24
- D.Platt, A. Raz, "Modulation of the lung colonization of B16-F1 melanoma cells by citrus pectin" Journal of the National Cancer Institut, vol.84, n.6, 438-442 (1992)
- F.Puglisi, A. M. Minisini, F. Barbone , D. Intersimone, G. Aprile, C.Puppin, G. Damante, I. Paron, G. Tell, A. Piga, C. Di Loreto, Cancer Letters, 212, (2004) 233-239
- R. Rebizak, M. Schaefer, E Dellacherie. Polymeric Conjugates of Gd3+-Diethylenetriaminepentaacetic Acid and Dextran. 2. Influence of Spacer Arm Length and Conjugate Molecular Mass on the Paramagnetic Properties and Some Biological Parameters. Bioconjugate Chemistry, 9 (1), 94 -99, (1998)
- M. F. Semmelhack, C. R. Schmid, D. A. Cortes, C. S. Chou. Oxidation of alcohols to aldehydes with oxygen and cupric ion, mediated by nitrosonium ion. Journal of the American Chemical Society, vol. 106, n°11, pp. 3374-3376, (1984)
- D. Shahbazi-Gahrouei, M. Williams, S. Rizvi, B.J. Allen. In vivo studies of Gd-DTPA monoclonal antibody and Molecular MRI With Targeted Contrast Agents 523 gd-porphyrins: Potential magnetic resonance imaging contrast agents for melanoma. J Magn Reson Imaging 14(2):169-74 (2001).

## Claims

1. A compound of formula (I) 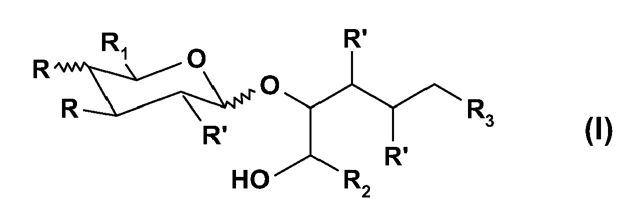 wherein:
**R** is, the same or different and each independently, a group selected from hydroxyl (-OH), acetate (-OCOCH₃), sulphate (-OSO₃H), phosphate (-OPO₃H₂), amino (-NH₂) or acetylamino (-NHCOCH₃); or one of the R groups is as above defined and the other is a straight or branched saccharidic or oligosaccharidic chain;
**R'** is, the same or different and each independently, a group selected from hydroxyl (-OH), acetate (-OCOCH₃), sulphate (-OSO₃H), phosphate (-OPO₃H₂), amino (-NH₂) or acetylamino (-NHCOCH₃);
**R₁** and **R₂** are, the same or different and each independently, hydroxymethyl (-CH₂OH), carboxyl optionally esterified with a straight or branched C₁-C₄ alkyl chain (-COOH, -COOAlk), a group of formula (II)
-CH₂-O-CH₂-CH(OH)-CH₂R₃ (II)
or a group -CH₂R₃;
**R₃** is, independently in each occurrence, a diagnostically or radiotherapeutically effective chelating moiety labelled with a paramagnetic metal ion or radionuclide, bearing a terminal amino group for its linkage (-NH-) with the rest of the molecule;
and the pharmaceutically acceptable salts thereof.

2. A compound of formula (I) according to claim 1 wherein both R groups are selected, each independently, from hydroxyl (-OH), acetate (-OCOCH₃), sulphate (-OSO₃H), phosphate (-OPO₃H₂), amino (-NH₂) or acetylamino (-NHCOCH₃).

3. A compound of formula (I) according to claim 2 wherein both R groups are hydroxyl (-OH) groups.

4. A compound of formula (I) according to claim 1 wherein one of the R groups is selected from hydroxyl (-OH), acetate (-OCOCH₃), sulphate (-OSO₃H), phosphate (-OPO₃H₂), amino (-NH₂) or acetylamino (-NHCOCH₃) and the other R group is a straight or branched saccharidic or oligosaccharidic chain selected from the group consisting of lactitol, lactosylamine, maltitol, maltosylamine, or modified citrus pectins.

5. A compound of formula (I) according to claim 4 wherein one of the R groups is hydroxyl (-OH) and the other R group has any one of the formulae (III) below 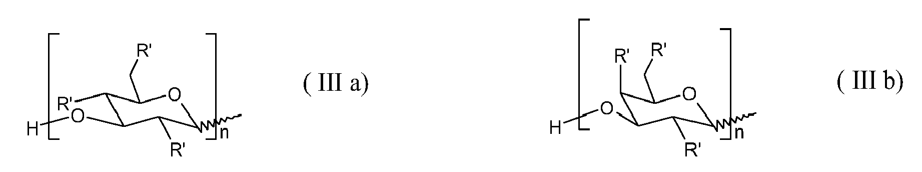 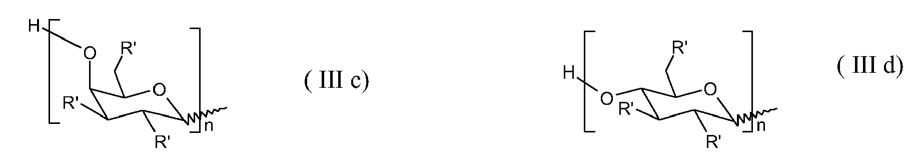 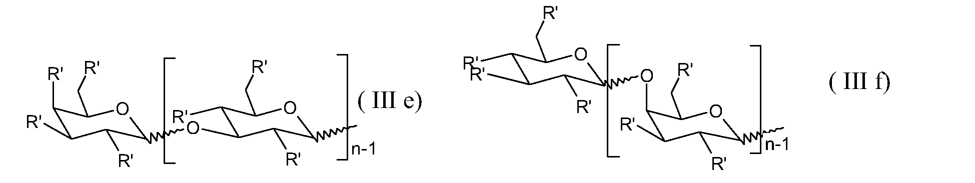 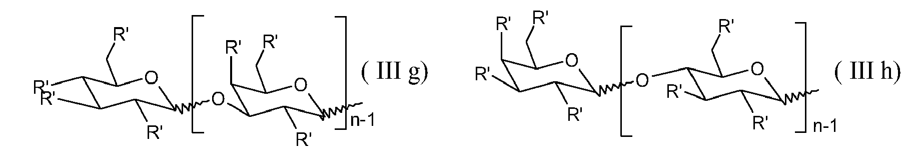 wherein R' is as defined in claim 1 and n is an integer from 1 to 7.

6. A compound of formula (I) according to claim 5 wherein R' is hydroxyl (-OH) and n is an integer from 1 to 3.

7. A compound of formula (I) according to any one of claims from 1 to 6 wherein both R₁ and R₂ are hydroxymethyl or optionally esterified carboxyl groups.

8. A compound of formula (I) according to any one of claims from 1 to 6 wherein one of R₁ or R₂ is hydroxymethyl or optionally esterified carboxyl and the remaining one of R₁ and R₂ is a group -CH₂-O-CH₂-CH(OH)-CH₂R₃ (II) or -CH₂R₃, wherein R₃ is as defined in claim 1.

9. A compound of formula (I) according to any one of claims from 1 to 6 wherein R₁ and R₂ are selected, each independently, from -CH₂-O-CH₂-CH(OH)-CH₂R₃ (II) or -CH₂R₃, wherein R₃ is as defined in claim 1.

10. A compound of formula (I) according to any one of claims from 1 to 9 wherein R₃ is a diagnostically or radiotherapeutically effective chelating moiety, properly labelled with a paramagnetic metal ion or radionuclide, bearing a terminal amino group for its linkage (-NH-) with the rest of the molecule, selected from the group consisting of (DTPA), benzo-DTPA, dibenzo-DTPA, phenyl-DTPA, diphenyl-DTPA, benzyl-DTPA, dibenzyl-DTPA, (DTPA-BMA), (EOB-DTPA), (BOPTA), (DTPA-BMA), (DTPA-GLU), (DTPA-Lys), (EDTA), (DO3A) (DOTA), (HPDO3A), (AAZTA), (MCTA), (DOTMA), (DPDP), (EDTP), 1,4,7,10-tetraazacyclotetradecane-1,4,7,10-tetrakis[methylphosphonic] acid, 1,4,7,10-tetraazacyclotetradecane-1,4,7,10-tetrakis[methylene-(methylphosphinic)] acid, terpyridine, HYNIC, TETA, (BAT), (MAG3), MAMA, DADS, CODADS and derivatives thereof.

11. A compound of formula (I) according to claim 10 wherein R₃ is a diagnostically effective chelating moiety selected from

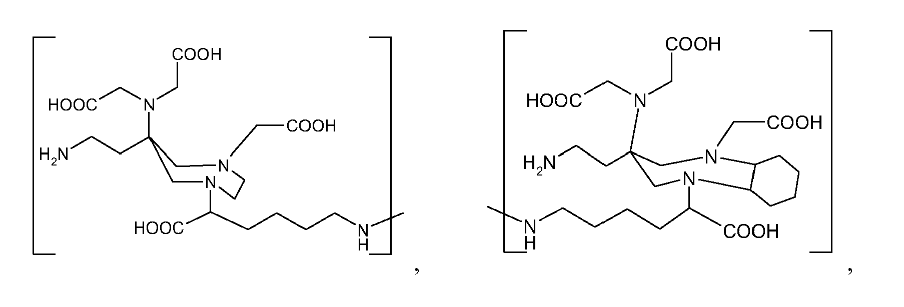

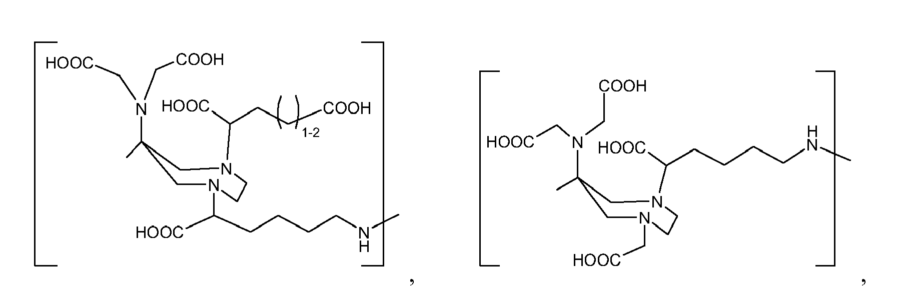

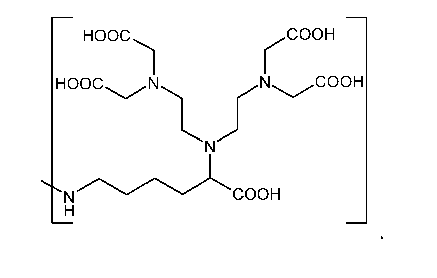

12. A compound of formula (I) according to any one of claims from 1 to 10 wherein R₃ is a diagnostically effective chelating moiety labelled with a paramagnetic metal ion selected from Fe(2+), Fe(3+), Cu(2+), Ni(2+), Rh(2+), Co(2+), Cr(3+), Gd(3+), Eu(3+), Dy(3+), Tb(3+), Pm(3+), Nd(3+), Tm(3+), Ce(3+), Y(3+), Ho(3+), Er(3+), La(3+), Yb(3+), Mn(3+) or Mn(2+).

13. A compound of formula (I) according to claim 12 wherein R₃ is a diagnostically effective chelating moiety labelled with Gd(3+).

14. A compound of formula (I) according to any one of claims from 1 to 10 wherein R₃ is a diagnostically effective chelating moiety labelled with a radionuclide selected from 64Cu, 67Ga, 68Ga, 99mTc, and 111In.

15. A compound of formula (I) according to any one of claims from 1 to 10 wherein R₃ is a radiotherapeutically effective chelating moiety labelled with a radionuclide selected from 64Cu, 90Y, 105Rh, 111In, 117mSn, 149Pm, 153Sm, 161Tb, 166Dy, 166Ho, 175Yb, 177Lu, 186/188Re, 199Au, and 159Gd.

16. A compound of formula (I) selected from 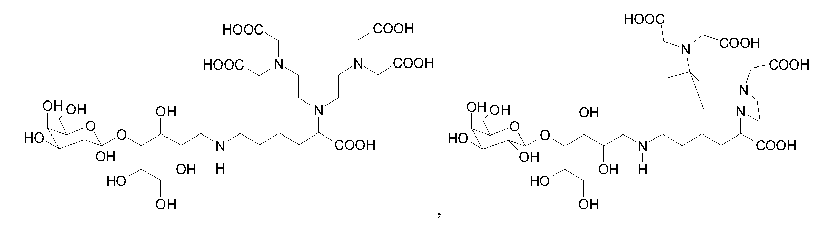 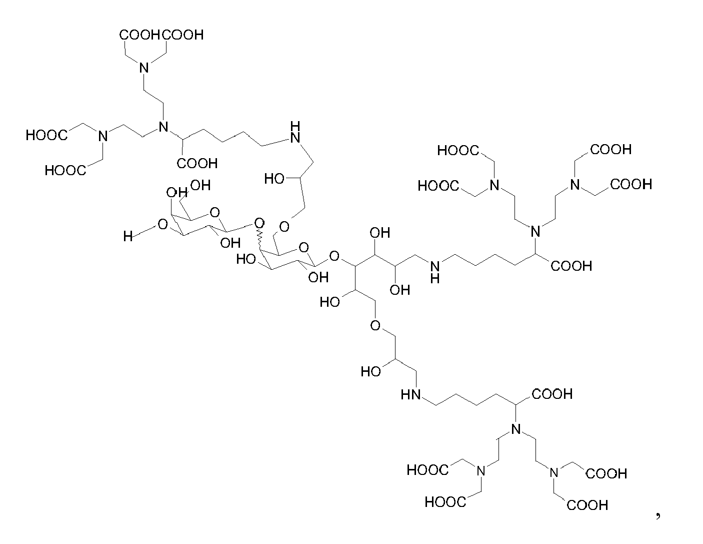 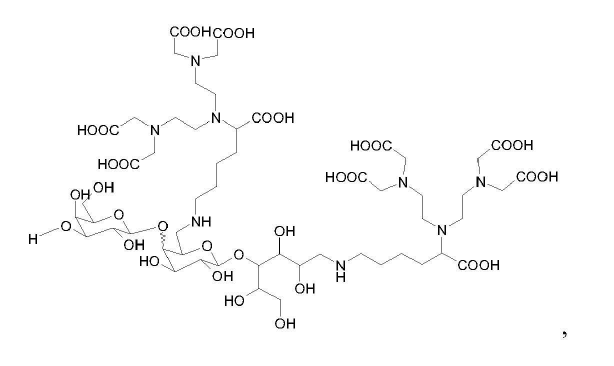 labelled with a paramagnetic metal ion as defined in claim 12.

17. A compound of formula (I) according to claim 16 labelled with Gd(3+).

18. A process for the preparation of a compound of formula (I) as defined in claim 1 which process comprises:
a) reacting under reductive conditions, in a suitable solvent and in presence of an acidifying agent, a saccharide or oligosaccharide of formula (IV) 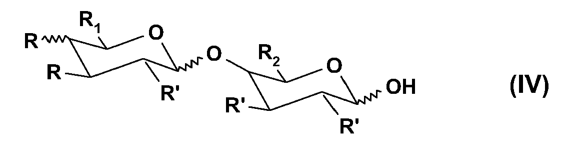 wherein R, R', R₁ and R₂ are as defined in claim 1 or R₁ and R₂ additionally represent, each independently, an aldehyde group (-CHO); with a compound of formula (V)
**R₃-H** **(V)**
wherein R₃ is the chelating moiety in unlabelled form, as defined in claim 1, so as to obtain a compound of formula (I') 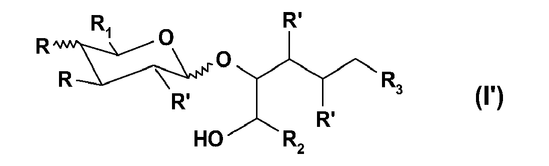 wherein R, R', R₁ and R₂ have the above reported meanings and R₃ is the above chelating unit; and
b) labelling the resulting compound of formula (I') with a paramagnetic metal ion or a radionuclide so as to obtain the compound of formula (I) and, optionally, converting it into a pharmaceutically acceptable salt thereof.

19. A process according to claim 18 wherein step (a) is carried out in the presence of a reducing agent selected from sodium cyanoborohydride, sodium tetraborate, lithium aluminium hydride, sodium triacetoxyborohydride and α-picoline-borane.

20. A compound of formula (I') 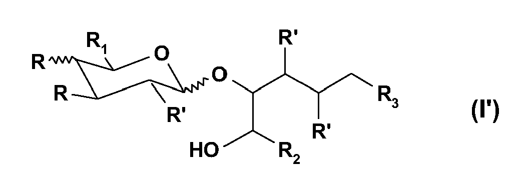 wherein R, R', R₁ and R₂ are as defined in claim 1 and R₃ is, independently in each occurrence, a diagnostically or radiotherapeutically effective chelating moiety bearing a terminal amino group for its linkage (-NH-) with the rest of the molecule.

21. A compound of formula (I) according to claim 1 for use as a diagnostic or radiotherapeutic agent.

22. Use of a compound of formula (I) according to claim 1 in the preparation of a diagnostic or radiotherapeutic agent selectively binding to lectins.

23. A pharmaceutical compositions comprising, as an active ingredient, at least one compound of formula (I) according to claim 1, including pharmaceutically acceptable salts thereof, in combination with one or more pharmaceutically acceptable carriers or excipients.
